# EUROPÄISCHE PATENTSCHRIFT

(11) **EP 1 083 988 B1**
(45) Veröffentlichungstag und Bekanntmachung des Hinweises auf die Patenterteilung: **21.04.2004**
(21) Anmeldenummer: 98965732.5
(22) Anmeldetag: 02.12.1998
(51) Int. Cl.: B01J 23/54, B01J 23/66, C07C 67/055

(54) **KATALYSATOR AUF DER BASIS VON PALLADIUM, GOLD, ALKALI UND LANTHANOID UND VERFAHREN ZUR HERSTELLUNG VON VINYLACETAT**
CATALYST BASED ON PALLADIUM, GOLD, ALKALI, AND LANTHANOID, AND METHOD FOR PRODUCING VINYL ACETATE
CATALYSEUR A BASE DE PALLADIUM, D'OR, DE METAL ALCALIN ET D'UN LANTHANIDE ET PROCEDE DE PREPARATION D'ACETATE DE VINYLE

(30) Priorität: 11.12.1997 DE 19755023
(43) Veröffentlichungstag der Anmeldung: 21.03.2001
(73) Patentinhaber: Celanese Chemicals Europe GmbH, 60439 Frankfurt am Main (DE); Celanese International Corporation, Dallas, Texas 75381 (US)
(72) Erfinder: HERZOG, Bernhard, D-46145 Oberhausen (DE); WANG, Tao, Corpus Christi, TX 78413 (US); NICOLAU, Ioan, Corpus Christi, TX 78413 (US)
(74) Vertreter: Gille Hrabal Struck Neidlein Prop Roos
(86) Internationale Anmeldenummer: PCT/EP1998/007816
(87) Internationale Veröffentlichungsnummer: WO 1999/029418

(56) Entgegenhaltungen:
- EP-A- 0 723 810
- EP-A- 0 723 811
- WO-A-99/22683
- US-A- 5 591 688

## Beschreibung

Die vorliegende Erfindung betrifft einen Katalysator, der Palladium und/oder dessen Verbindungen, Gold und/oder dessen Verbindungen, Alkalimetallverbindungen und mindestens ein spezifisches weiteres Metall und/oder dessen Verbindungen enthält, und seine Verwendung zur Herstellung von Vinylacetat aus Essigsäure, Ethylen und Sauerstoff oder Sauerstoff enthaltenden Gasen.

Es ist bekannt, daß man in der Gasphase Ethylen mit Essigsäure und Sauerstoff oder sauerstoffhaltigen Gasen an Palladium/Gold/Alkalimetall enthaltenden Festbett-Katalysatoren zu Vinylacetat umsetzen kann.

Die Palladium/Gold/Alkalimetall enthaltenden Katalysatoren weisen eine besondere Edelmetallverteilung auf, bei der die Edelmetalle in einer Schale auf den Trägerteilchen vorliegen, während der Kern der Teilchen weitgehend frei von Edelmetallen ist. Katalysatoren mit dieser Edelmetallverteilung zeichnen sich durch eine erhöhte spezifische Leistung (g Vinylacetat/g Edelmetall) aus. Die Edelmetallverbindung in Schalenform erreicht man durch Imprägnieren und anschließendes Ausfällen der Edelmetalle mit alkalischen Verbindungen.

In dem in US-A-4 048 096 offenbarten Verfahren zur Herstellung von Palladium, Kalium und Gold enthaltenden Katalysatoren, wird zunächst das Trägermaterial mit einer wäßrigen Lösung, die eine Mischung aus Palladium und Goldsalzen enthält, imprägniert. Anschließend werden die Metallsalze durch Behandlung mittels Alkalien in wasserunlösliche Verbindungen überführt und auf diese Weise auf dem Trägermaterial fixiert. Durch nachfolgende Behandlung mit einem Reduktionsmittel werden die Palladium- und Goldverbindungen zu den entsprechenden Metallen reduziert. Abschließend wird das mit Palladium und Gold beladene Trägermaterial mit einer Alkalimetallacetatlösung behandelt und getrocknet. Der Imprägnierungsschritt mit der wäßrigen Palladium- und Goldsalze enthaltenden Lösung ist dadurch gekennzeichnet, daß das Volumen der Imprägnierungslösung dem Porenvolumen des Trägermaterials entspricht. Der erhaltene Katalysator weist eine Schalenstruktur auf, in der Palladium und Gold in einer Schalendicke von etwa 0,5 Millimetern über die Oberfläche des Trägermaterials verteilt sind.

Auch US-A-3 775 342 offenbart ein Verfahren zur Herstellung von Palladium, Kalium und Gold enthaltenden Katalysatoren durch Imprägnierung mit einer Lösung von Palladium- und Goldsalzen, durch anschließende Behandlung mit einer Alkalilösung, die dazu führt, daß sich wasserunlösliche Palladium- und Goldverbindungen auf dem Träger niederschlagen, und durch anschließende Reduktion der Metallverbindungen zu den entsprechenden Edelmetallen. Eine Behandlung des Trägermaterials mit einer Alkalimetallacetatlösung kann vor oder nach dem Reduktionsschritt erfolgen.

In US-A-5 185 308 wird ein Palladium, Kalium und Gold enthaltender Schalenkatalysator offenbart, in dem die Edelmetalle in einer Schalendicke von 1 Millimeter über das Trägermaterial verteilt sind. Der bekannte Katalysator wird durch ein Gewichtsverhältnis von Gold zu Palladium in dem Bereich von 0,6 bis 1,25 charakterisiert.

Weiter ist die Herstellung eines Palladium, Kalium und Gold enthaltenden Schalenkatalysators bekannt, wobei ein mit einem Bindemittel, beispielsweise einem Alkali- oder Erdalkalicarboxylat, versehenes Trägermaterial vor der Imprägnierung mit einer Säure gewaschen und nach der Imprägnierung mit einer Base behandelt wird (EP-A-0 519 435).

Die EP-A-0 723 810 offenbart ein Verfahren zur Herstellung von Vinylacetat unter Verwendung eines Katalysators enthaltend Pd, Au, K und ein zusätzliches Element der Gruppen IA, IIA, IIIA und IVB des Periodensystems.

Die WO-A-99/22683, die Stand der Technik gemäß Artikel 54(3) EPÜ darstellt, offenbart ein Verfahren zur Herstellung von Vinylacetat in der Gasphase aus Ethylen, Essigsäure und Sauerstoff an einem Katalysator, der Palladium, Gold und ein drittes Metall ausgewählt aus Magnesium, Calcium, Barium, Zirkonium und Cer auf einem Träger enthält. Im Verlauf seiner Herstellung wird der Katalysator mit geeigneten Alkaliverbindungen behandelt.

In dem in US-A-5 332 710 offenbarten Verfahren zur Herstellung eines Palladium, Gold und Kalium enthaltenden Schalenkatalysators wird der mit einer wäßrigen Palladium und Goldsalzlösung imprägnierte Träger in eine Natriumhydroxid oder Kaliumhydroxid enthaltende wäßrige Fixierlösung eingetaucht und darin wenigstens 0,5 h lang bewegt.

Überraschenderweise wurde nun gefunden, daß derartige Katalysatoren durch Zusatz von mindestens einem weiteren spezifischem Metall und/oder einer Verbindung des Metalls deutlich verbessert werden, d.h. eine höhere Raum-Zeit-Ausbeute bei gleicher oder höherer Selektivität zu Vinylacetat bewirken.

Ein Gegenstand der Erfindung ist demgemäß ein Verfahren zur Herstellung von Vinylacetat in der Gasphase aus Ethylen, Essigsäure und Sauerstoff oder Sauerstoff enthaltenden Gasen an einem Katalysator, der 0,5-2,0 Gew.-% Palladium und/oder dessen Verbindungen, 0,2-1,3 Gew.-% Gold und/oder dessen Verbindungen sowie 0,3-10 Gew.-% Alkalimetallverbindungen auf einem Träger enthält, dadurch gekennzeichnet, daß der Katalysator zusätzlich 0,01-1 Gew.-% mindestens ein weiteres Metall, das aus der Gruppe ausgewählt wird, die aus Praseodym, Neodym, Promethium, Samarium, Europium, Gadolinium, Terbium, Dysprosium, Holmium, Erbium, Thulium, Ytterbium und Lutetium, Scandium, Yttrium und Lanthan besteht, und/oder dessen Verbindungen enthält, wobei sich die Prozentangaben auf die Metallgehalte, bezogen auf die Gesamtmasse des Katalysators, beziehen.

Ein weiterer Gegenstand der Erfindung ist ein Katalysator, der 0,5-2,0 Gew.-% Palladium und/oder dessen Verbindungen, 0,2-1,3 Gew.-% Gold und/oder dessen Verbindungen sowie 0,3-10 Gew.-% Alkalimetallverbindungen auf einem Träger enthält, dadurch gekennzeichnet, daß der Katalysator zusätzlich 0,01-1 Gew.-% mindestens ein weiteres Metall, das aus der Gruppe ausgewählt wird, die aus Praseodym, Neodym, Promethium, Samarium, Europium, Gadolinium, Terbium, Dysprosium, Holmium, Erbium, Thulium, Ytterbium und Lutetium, Scandium, Yttrium und Lanthan besteht, und/oder dessen Verbindungen enthält, wobei sich die Prozentangaben auf die Metallgehalte, bezogen auf die Gesamtmasse des Katalysators, beziehen.

Zur Herstellung der erfindungsgemäßen Katalysatoren wird vorzugsweise wie folgt vorgegangen (US-A-3 775 342, US-A-4 048 096, US-A-5 332 710):
(1) Zunächst werden die Trägerteilchen unter inniger Durchmischung mit mindestens einer Lösung von mindestens einem Salz der Elemente Palladium und Gold sowie von mindestens einem Salz von mindestens einem genannten, weiteren Metall ein- oder mehrmals getränkt.
(2) Der vorbehandelte Träger wird mit einer alkalisch reagierenden Fixierlösung behandelt, was dazu führt, daß die Edelmetalle und die genannten, weiteren Metalle in Form von wasserunlöslichen Verbindungen auf der Oberfläche der Trägerteilchen ausgefällt werden und so fixiert werden.
(3) Durch Behandlung mit einem Reduktionsmittel werden die auf den Trägerteilchen abgeschiedenen Edelmetallverbindungen zu den entsprechenden Metallen reduziert. Auf diese Weise bildet sich auf der Oberfläche der Trägerteilchen eine mit mindestens einem der genannten weiteren Metalle dotierte Edelmetallschale aus.
(4) Durch Waschen des behandelten Katalysators werden störende Anionen entfernt.
(5) Es erfolgt die Trocknung des behandelten Katalysators bei höchstens 150°C.
(6) Der getrocknete Träger wird mit einer Lösung, die mindestens eine Alkalimetallverbindung enthält, behandelt.
(7) Abschließend wird der behandelte Träger bei höchstens 150°C getrocknet.

In der Arbeitsweise nach Schritt (1) kann auch in der weise vorgegangen werden, daß die katalytisch aktiven Substanzen enthaltenden Salzlösungen durch ein- oder mehrmaliges Aufsprühen, Aufdampfen oder Tauchen auf dem Träger aufgebracht werden.

Unter dem Begriff "weiteres Metall" werden Cer, Praseodym, Neodym, Promethium, Samarium, Europium, Gadolinium, Terbium, Dysprosium, Holmium, Erbium, Thulium, Ytterbium, Lutetium, Scandium, Yttrium und Lanthan verstanden.

Als Träger eignen sich die bekannten inerten Trägermaterialien wie Kieselsäure, Aluminiumoxid, Alumosilikate, Silikate, Titanoxid, Zirkonoxid, Titanate, Siliciumcarbid und Kohle. Besonders geeignet sind Träger dieser Art mit einer spezifischen Oberfläche von 40 bis 350 m²/g (gemessen nach der BET-Methode) und einem mittleren Porenradius von 50 bis 2000 Å (Ångström) (gemessen mit Quecksilber-Porosimetrie), vor allem Kieselsäure (SiO₂) und SiO₂-Al₂O₃-Gemische. Diese Träger können in jedweder Form, wie z.B. in Form von Kugeln, Tabletten, Ringen, Sternen oder anders geformten Teilchen benutzt werden, deren Durchmesser bzw. deren Länge und Dicke im allgemeinen bei 3 bis 9 mm liegt.

Solche Träger können z.B. aus aerogenem SiO₂ oder einem aerogenen SiO₂-Al₂O₃-Gemisch hergestellt werden, die beispielsweise durch Flammenhydrolyse von Siliciumtetrachlorid oder eines Siliciumtetrachlorid-Aluminiumtrichlorid-Gemisches in einer Knallgasflamme hergestellt werden können (US-A-3 939 199).

Als Lösungsmittel für die Palladium-, Gold-, Alkalimetall-Salze und die weiteren Metallsalze sind alle Verbindungen geeignet, in denen die gewählten Salze löslich sind und die nach der Imprägnierung leicht wieder durch Trocknung zu entfernen sind. Geeignet sind für die Acetate vor allem unsubstituierte Carbonsäuren mit 2 bis 10 Kohlenstoffatomen, wie Essigsäure, Propionsäure, n- und iso-Buttersäure und die verschiedenen Valeriansäuren. Wegen ihrer physikalischen Eigenschaften und auch aus wirtschaftlichen Gründen wird Essigsäure unter den Carbonsäuren bevorzugt. Für die Chloride, Chloro- und Acetatokomplexe ist vor allem Wasser geeignet. Die zusätzliche Verwendung eines weiteren Lösungsmittels ist dann zweckmäßig, wenn die Salze in der Essigsäure oder im Wasser nicht genügend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, aber auch Kohlenwasserstoffe wie Benzol.
Man kann mehrere Salze von Palladium, Gold, Alkalimetall und dem jeweiligen weiteren Metall aufbringen, aber im allgemeinen bringt man von jedem dieser Elemente genau ein Salz auf.
Die in der Arbeitsweise nach Schritt (1) jeweils aufzubringenden Elemente Palladium und Gold sowie das jeweils aufzubringende weitere Metall können in Form von Salzlösungen einzeln oder auch in beliebiger Kombination in einer beliebigen Reihenfolge aufgebracht werden, vorzugsweise verwendet man eine einzige Lösung, die diese aufzubringenden Elemente in Form von Salzen enthält. Besonders bevorzugt ist der Einsatz einer einzigen Lösung, die von jedem dieser aufzubringenden Elemente genau ein Salz enthält.

Bevorzugt enthält diese Lösung ein Salz eines einzigen weiteren Metalles, man kann aber auch eine Lösung verwenden, die je ein Salz verschiedener der weiteren Metalle enthält.

Wenn im folgenden im allgemeinen über "die Lösung der Salze" gesprochen wird, so gilt sinngemäß dasselbe für den Fall, daß mehrere Lösungen der Reihe nach eingesetzt werden, die jeweils nur einen Teil der insgesamt aufzubringenden Salze enthalten, wobei sich die einzelnen Teile zur Gesamtmenge der Salze ergänzen, die auf den Träger aufgebracht werden sollen.

Zur Durchführung der Arbeitsweise nach Schritt (1) wird die Lösung der Salze auf die Trägerteilchen aufgebracht, indem man diese ein- oder mehrmals mit dieser Lösung tränkt, wobei das gesamte Volumen der Lösung auf einmal oder in zwei oder mehr Teilvolumina unterteilt eingesetzt wird. Zweckmäßig ist es jedoch, das gesamte Volumen der Salzlösung auf einmal zu verwenden, so daß durch einmaliges Tränken die Trägerteilchen mit der gewünschten Menge an den aufzubringenden Elementen imprägniert werden, wobei sich die Trocknung sofort anschließen kann. Beim Tränken der Reihe nach mit mehreren Teilvolumina wird nach jedem Tränken sofort getrocknet.

Die "sofortige" Trocknung bedeutet dabei, daß zügig mit der Trocknung der getränkten Teilchen begonnen werden muß. Dabei reicht es im allgemeinen, wenn nach dem Ende einer Tränkung spätestens nach 1/2 Stunde mit der Trocknung der Teilchen begonnen wird.

Die Tränkung der Trägerteilchen mit der Lösung der aufzubringenden Salze wird so vorgenommen, daß die Trägerteilchen mit der Lösung überschichtet und gegebenenfalls überschüssige Lösung dann abgegossen oder abfiltriert wird. Mit Rücksicht auf Lösungsverluste ist es vorteilhaft, nur die dem integralen Porenvolumen des Katalysatorträgers entsprechende Menge an Lösung einzusetzen.

Es ist zweckmäßig, während des Tränkens die Trägerteilchen innig zu durchmischen, beispielsweise in einem sich drehenden oder bewegten Kolben oder einer Mischtrommel, wobei sich die Trocknung sofort anschließen kann. Die Drehgeschwindigkeit oder Intensität der Bewegung muß einerseits groß genug sein, um eine gute Durchmischung und Benetzung der Trägerteilchen zu gewährleisten, andererseits darf sie nicht so hoch sein, daß es zu wesentlichem Abrieb am Trägermaterial kommt.

Die Lösung der Salze sollte eine Temperatur haben, die hoch genug ist, um ein Ausfallen der Salze während des Aufbringens auf den Träger zu verhindern. Die Temperatur sollte aber im allgemeinen nicht wesentlich über 70°C liegen, um eine zu starke Verdunstung des Lösungsmittels und eine Zersetzung der Edelmetallverbindungen zu verhindern.

Durch die Behandlung der gemäß Arbeitsschritt (1) getränkten Trägerteilchen mit einer alkalisch reagierenden Lösung werden die Salze der aufgebrachten Elemente in wasserunlösliche Verbindungen überführt und so auf der Trägeroberfläche fixiert (Arbeitsschritt (2)).

Als Fixierlösungen lassen sich beispielsweise wäßrige alkalisch reagierende Lösungen verwenden. Beispiele für derartige Lösungen sind wäßrige Lösungen von Alkalimetallsilikaten, Alkalimetallcarbonaten und -hydrogencarbonaten oder Alkalimetallhydroxiden.

Bevorzugt ist eine wässrige Lösung der Alkalihydroxide, insbesondere Kalium- oder Natriumhydroxid. Auch wässrige Lösungen, die Borverbindungen enthalten, können als alkalisch reagierende Lösungen verwendet werden. Hierbei sind besonders wässrige Lösungen von Borax, Kaliumtetraborat oder Mischungen aus Alkalilauge und Borsäure geeignet. Die alkalische Lösung kann Puffereigenschaften aufweisen.

Die Menge der in der Fixierlösung enthaltenden alkalisch reagierenden Verbindung ist zweckmäßigerweise so bemessen, däss sie für die stöchiometrische Umsetzung der aufgebrachten Palladium-, Gold-Salze und der Salze der genannten weiteren Metalle zu wasserunlöslichen Verbindungen mindestens ausreichend sind.

Es kann aber auch ein Überschuss an der in der Fixierlösung enthaltenen alkalisch reagierenden Verbindung angewandt werden, der Überschuss liegt im allgemeinen bei der 1- bis 10-fachen Menge, bezogen auf die stöchiometrisch erforderliche Menge.

Das Volumen der Fixierlösung ist wenigstens so zu bemessen, dass es ausreicht, den imprägnierten Träger vollständig mit der Fixierlösung zu bedecken. Vorzugsweise erfolgt die Fixierung nach der aus US-A-5,332,710 bekannten "Rotation-Immersion"-Technik, auf die hiermit direkt Bezug genommen wird ("incorporation by reference"). Diese Technik ist dadurch charakterisiert, dass der mit der Fixierlösung vollständig bedeckte Träger ab dem Beginn der Behandlung mit der Fixierlösung rotierend bewegt wird.

Jede Art von Rotation oder ähnlicher Behandlung, die die Trägerteilchen in Bewegung hält, kann benutzt werden, da die genaue Art und Weise nicht kritisch ist. Wichtig ist jedoch die Intensität der Bewegung. Diese soll ausreichen, die gesamte Oberfläche der imprägnierten Träger gleichmäßig mit der alkalischen Fixierlösung zu benetzen.

Danach beläßt man den behandelten Träger bis zu 16 Stunden bei Raumtemperatur ruhend in der Fixierlösung, um sicherzustellen, daß die aufgebrachten Palladium, Gold-Salze und die Salze der genannten weiteren Metalle vollständig auf dem Katalysatorträger in Form von wasserunlöslichen Verbindungen ausgefällt werden.

Die Umsetzung auf dem Träger kann aber auch bei erhöhter Temperatur, z.B. bei 70°C durchgeführt werden.

Nach beendeter Fixierung wird die überstehende Fixierlösung abgegossen. Gegebenenfalls kann sich nun eine Wäsche des behandelten Trägers anschließen, um die auf dem behandelten Träger enthaltenen löslichen Verbindungen, z.B. die aus dem Fixierungsschritt freigesetzten Alkalimetallchloride und einen gegebenenfalls vorhandenen Überschuß an der in der Fixierlösung enthaltenen alkalisch reagierenden Verbindung durch Waschen zu entfernen.

Dazu wird der behandelte Träger bei Raumtemperatur mit der Waschflüssigkeit, vorzugsweise mit fließendem, demimeralisiertem Wasser kontinuierlich gewaschen. Die Wäsche wird fortgesetzt, bis störende Anionen, z.B. Chloride vom Träger weitgehend entfernt sind.

Anschließend kann der feuchte imprägnierte Katalysator träger getrocknet werden, was zweckmäßig ist, wenn die nachfolgende Reduktion der abgeschiedenen Edelmetallverbindungen zu den entsprechenden Metallen (Arbeitsschritt (3)) in der Gasphase durchgeführt wird.

Die Reduktion der auf dem Katalysatorträger fixierten wasserunlöslichen Verbindungen zu den entsprechenden Metallen kann mit einem gasförmigen Reduktionsmittel durchgeführt werden (Arbeitsschritt (3)). Die Reduktionstemperatur liegt im allgemeinen zwischen 40 und 260°C, vorzugsweise zwischen. 70 und 200°C. Im allgemeinen ist es zweckmäßig, für die Reduktion ein mit Inertgas verdünntes Reduktionsmittel zu verwenden, das 0,01 bis 50 Vol.-%, vorzugsweise 0,5 bis 20 Vol.-% Reduktionsmittel enthält. Als Inertgas kann beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen oder andere Olefine in Frage. Die Reduktion kann auch in flüssiger Phase bei einer Temperatur von 0°C bis 90°C, vorzugsweise von 15 bis 25°C durchgeführt werden. Als Reduktionsmittel lassen sich beispielsweise wäßrige Lösungen von Hydrazin, Ameisensäure oder Alkaliborhydriden, vorzugsweise Natriumborhydrid, verwenden. Die Menge des Reduktionsmittels richtet sich nach der Menge der Edelmetalle; das Reduktionsäquivalent soll mindestens das einfache an Oxidationsäquivalent betragen, jedoch schaden größere Mengen Reduktionsmittel nicht.
Bei dem Reduktionsschritt ist es wesentlich, die Reduktionsbedingungen so zu wählen, daß eine Reduktion der fixierten wasserunlöslichen Edelmetallverbindungen zu den entsprechenden Edelmetallen erfolgt. Hingegen ist es unwesentlich, ob unter den gewählten Reduktionsbedingungen auch die fixierten wasserunlöslichen Verbindungen der weiteren Metalle in die entsprechenden Metalle überführt werden, da es für die Eignung der erfindungsgemäßen Katalysatoren zur Vinylacetatherstellung nicht kritisch ist, ob in der Edelmetallschale der erfindungsgemäßen Katalysatoren die weiteren Metalle als Elemente und/oder als deren Verbindungen vorliegen.

Ist der Waschschritt nach der beendeten Fixierung (Arbeitsschritt (2)) nicht erfolgt oder erfolgt die Reduktion mit Hilfe einer wäßrigen Lösung eines Reduktionsmittels, muß nach beendeter Reduktion der behandelte Katalysatorträger zur Entfernung störender Verbindungen, z.B. zur Entfernung von Chloridresten, die vom Imprägnierungsschritt herrühren, und die durch die Fixierung und Reduktion der Edelmetalle freigesetzt werden, mehrmals gewaschen werden. (Arbeitsschritt (4)).

Dazu wird der behandelte Träger bei Raumtemperatur mit der Waschflüssigkeit, vorzugsweise mit fließendem, demineralisiertem Wasser kontinuierlich gewaschen, bis störende Anionen, z.B. Chloride, entfernt sind.

Falls in Arbeitsschritt (3) mit einer wäßrigen Lösung eines Reduktionsmittels gearbeitet wird, lassen sich mit dem Waschvorgang auch Reste des verwendeten Reduktionsmittels entfernen.

Anschließend wird der Katalysator bei Temperaturen von höchstens 150°C getrocknet (Arbeitsschritt (5)).

Gemäß Arbeitsschritt (6) wird der getrocknete Katalysatorträger anschließend mit einer Lösung einer Alkalimetallverbindung ein- oder mehrmals behandelt, vorzugsweise getränkt, wobei das gesamte Volumen der Lösung auf einmal oder in Teilvolumina unterteilt eingesetzt wird. Zweckmäßig ist es jedoch, das gesamte Volumen der Lösung auf einmal zu verwenden, so daß durch einmaliges Tränken die Trägerteilchen mit den gewünschten Mengen an aufzubrinender Alkalimetallverbindung imprägniert werden. Das Lösungsvolumen der Alkalimetallverbindung liegt bei dem einmaligen oder bei einem mehrfachen Tränken im allgemeinen zwischen 60 und 110 %, vorzugsweise zwischen 80 und 100 % des Porenvolumens.

Die Lösung der Alkalimetallverbindung kann auch durch ein- oder mehrmaliges Aufsprühen, Aufdampfen oder Tauchen auf dem Träger aufgebracht werden.

Nach dem Behandeln mit einer Lösung einer Alkalimetallverbindung wird der Katalysatorträger abschließend bis höchstens 150°C getrocknet (Arbeitsschritt (7)).

Die Alkalimetallverbindung wird in einer solchen Menge verwendet, so daß der Katalysatorträger nach dem Trocknen 0,1 bis 10 Gew.-% an Alkalimetall enthält.

Die gemäß den Arbeitsschritten (5) und (7) durchzuführende Trocknung des behandelten Katalysatorträgers erfolgt in einem Heizluftstrom oder in einem Inertgasstrom, beispielsweise in einem Stickstoff- oder Kohlendioxidstrom. Die Temperatur bei der Trocknung sollte dabei im allgemeinen 60 bis -150°C, vorzugsweise 100 bis 150°C betragen. Gegebenenfalls wird dabei unter vermindertem Druck, im allgemeinen bei 0,01 MPa bis 0,08 MPa getrocknet.

Ist die Trocknung Bestandteil des Arbeitsschrittes (1) und gegebenenfalls der anderen Arbeitsschritte, so wird in der gleichen Weise verfahren.

Die fertigen Palladium, Gold, Alkalimetall und mindestens ein genanntes weiteres Metall enthaltenden Schalenkatalysatoren weisen folgende Metallgehalte auf:

| | |
|---|---|
| Palladiumgehalt | 0,5 - 2,0 Gew.-%, vorzugsweise 0,6 - 1,5 Gew.-%; |
| Goldgehalt | 0,2 - 1,3 Gew.-%, vorzugsweise 0,3 - 1,1 Gew.-%; |
| Alkalimetallgehalt | 0,3 - 10 Gew.-%, |

Vorzugsweise verwendet man Kalium.

| | |
|---|---|
| Kaliumgehalt | im allgemeinen 0,5 - 4,0 Gew.-%, vorzugsweise 1,5 - 3,0 Gew.-%; |
| Gehalt des weiteren Metalls | 0,01 - 1 Gew.-%, vorzugsweise 0,05 - 0,5 Gew.-%. |

Wird zur Dotierung der Palladium, Gold und Alkalimetall enthaltenden Schalenkatalysatoren mehr als ein genanntes weiteres Metall verwendet, wird unter dem Begriff "Gehalt des weiteren Metalls" der Gesamtgehalt aller in dem fertigen Katalysator enthaltenen weiteren Metalle verstanden. Die angegebenen Prozentzahlen betreffen stets die im Katalysator anwesenden Mengen der Elemente Palladium, Gold, Alkalimetall und des weiteren Metalls, bezogen auf die Gesamtmasse des Katalysators (aktive Elemente plus Anionen plus Trägermaterial).
Als Salze sind alle Salze von Palladium, Gold, einem Alkalimetall und des genannten weiteren Metalls geeignet, die löslich sind; bevorzugt sind die Acetate, die Chloride, die Acetato- und die Chlorokomplexe. Dabei muß aber im Fall störender Anionen, wie z.B. bei Chloriden, sichergestellt werden, daß diese Anionen vor dem Einsatz des Katalysators weitgehend entfernt werden. Dies geschieht durch Auswaschen des dotierten Trägers, z.B. mit Wasser, nachdem beispielsweise das als Chlorid aufgebrachte Palladium und Gold in eine unlösliche Form überführt wurden, etwa durch die Fixierung mit alkalisch reagierenden Verbindungen und/oder durch Reduktion (Arbeitsschritte (2) und (3)).

Als Salze von Palladium und Gold sind besonders geeignet Chlorid, Chlorokomplexe und Carboxylate, vorzugsweise die Salze der aliphatischen Monocarbonsäuren mit 2 bis 5 Kohlenstoffatomen, beispielsweise das Acetat, Propionat oder Butyrat. Weiter sind beispielsweise geeignet das Nitrat, Nitrit, Oxidhydrat, Oxalat, Acetylacetonat oder Acetoace tat. Wegen der guten Löslichkeit und Verfügbarkeit sind besonders die Chloride und Chlorokomplexe von Palladium und Gold bevorzugte Palladium- und Goldsalze.

Als Alkalimetallverbindung wird vorzugsweise mindestens eine Natrium-, Kalium-, Rubidium- oder Caesiumverbindung eingesetzt, insbesondere eine Kaliumverbindung. Geeignet als Verbindungen sind vor allem Carboxylate, insbesondere Acetate und Propionate. Geeignet sind auch Verbindungen, die unter den Reaktionsbedingungen in das Alkaliacetat übergehen, wie etwa das Hydroxid, das Oxid oder das Carbonat.

Als weitere Metallverbindung wird vorzugsweise mindestens eine Praseodym-, Neodym-, Samarium-, Europium- oder Dysprosiumverbindung eingesetzt. Man kann aber auch Gemische dieser Verbindungen einsetzen.

Als Verbindungen der weiteren Metalle sind vor allem die Chloride, Nitrate, Acetate und Acetylacetonate geeignet. Bei den erfindungsgemäßen Katalysatoren sind die Edelmetalle und die jeweiligen genannten weiteren Metalle und/oder deren Verbindungen in einer Schale auf dem Trägerteilchen aufgebracht.

Die Herstellung des Vinylacetats erfolgt im allgemeinen durch Leiten von Essigsäure, Ethylen und Sauerstoff enthaltenden Gasen bei Temperaturen von 100 bis 220°C, vorzugsweise 120 bis 200°C, und bei Drucken von 0,1 bis 2,5 MPa, vorzugsweise 0,1 bis 2,0 MPa, über den fertigen Katalysator, wobei nichtumgesetzte Komponenten im Kreis geführt werden können. Unter Umständen ist auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Mit Hilfe der erfindungsgemäßen Katalysatoren gelingt es, bei gleichen Reaktionsbedingungen im Vergleich zu den bekannten Katalysatoren mehr Vinylacetat pro Reaktorvolumen und Zeit bei gleichzeitig verbesserter Selektivität herzustellen.

Dadurch wird die Aufarbeitung des erhaltenen Rohvinylacetats erleichtert, da der Vinylacetatgehalt im Reaktorausgangsgas höher ist, was weiterhin zu einer Energieersparnis im Aufarbeitungsteil führt. Eine geeignete Aufarbeitung wird z.B. in der US-A-5 066 365 beschrieben.

Will man dagegen die Raum-Zeit-Ausbeute konstant halten, so kann man die Reaktionstemperatur senken und dadurch bei gleicher Gesamtleistung die Reaktion selektiver durchführen, wobei Edukte eingespart werden. Dabei wird auch die Menge des als Nebenprodukt entstehenden und daher auszuschleusenden Kohlendioxids und der mit dieser Ausschleusung verbundene Verlust an mitgeschlepptem Ethylen geringer. Darüber hinaus führt diese Fahrweise zu einer Verlängerung der Katalysatorstandzeit.

Die folgenden Beispiele sollen die Erfindung erläutern, schränken sie aber nicht ein. Die Prozentangaben der Elemente Palladium, Gold, Kalium und des weiteren Metalls sind Gewichtsprozente, bezogen auf die Gesamtmasse des Katalysators.

Als Katalysatorträger wurde der von der Firma Süd-Chemie angebotene SiO₂-Träger mit der Bezeichnung KA 160 in Form von Kugeln mit einem Durchmesser von 5 mm verwendet. Das Porenvolumen von 1 l Träger war 335 ml.

### Beispiel 1

5,37 g (= 0,0164 mol) Kaliumtetrachloropalladat, 3,36 g (0,0089 mol) Kaliumtetrachloroaurat und 0,74 g (0,0018 mol) Praseodymtrinitrat-Pentahydrat wurden zusammen eingewogen und in 90 ml demineralisiertem Wasser (Lösungsvolumen = 100 % des Porenvolumens) gelöst. Unter leichter Bewegung wurde diese Lösung bei Raumtemperatur auf 147,5 g des Trägermaterials vollständig aufgesogen. Für die Ausfällung von unlöslichen Palladium-, Gold- und Praseodym-Verbindungen, die zur Ausbildung einer Edelmetallschale führt, wurde der vorbehandelte Träger mit einer Lösung von 3,1 g Natriumhydroxid in 300 ml demineralisiertem Wasser versetzt. Sofort nach Zugabe der alkalischen Fixierlösung wurde der Träger über einen Zeitraum von 2,5 Stunden am Rotationsverdampfer bei einer Rotationsgeschwindigkeit von 5 Umdrehungen pro Minute (UpM) bewegt. Zur Vervollständigung der Ausfällung wurde der Ansatz über einen Zeitraum von 14 Stunden bei Raumtemperatur stehengelassen. Anschließend wurde die überstehende Lösung abgegossen und der Ansatz mit demineralisiertem Wasser chloridfrei gewaschen. Dazu wurde eine Wasserflußrate von 200 ml/Minute über ungefähr 5 Stunden benötigt. Zur Überprüfung auf Chloridfreiheit wurde das Waschwasser mit einer Silbernitratlösung versetzt, und es wurde auf Silberchloridfällung geprüft. Im Anschluss wurde der Katalysator bei einer Temperatur von 100 DEG C über einen Zeit-raum von 2 Stunden getrocknet. Anschließend erfolgte die Reduktion mit einem Gasgemisch bestehend aus 5 Vol.-% Ethylen und 95 Vol.-% Stickstoff, indem dieses Gasgemisch über einen Zeitraum von 5 Stunden bei einer Temperatur von 150 °C über den Katalysator geleitet wurde. Anschließend wurde der reduzierte Katalysator mit einer Lösung von 10 g Kaliumacetat in 75 ml demineralisiertem Wasser (Lösungsvolumen = 83 % des Porenvolumens) portionsweise getränkt und bei einer Temperatur von 100 °C über einen Zeitraum von 2 Stunden mit Heißluft getrocknet.

Der fertige Katalysator enthielt 1,1 Gew.-% Pd, 1,1 Gew.-% Au, 2,5 Gew.-% K und 0,16 Gew.-% Pr.

### Beispiel 2

Es wurde analog der Arbeitsweise nach Beispiel 1 vorgegangen, als Verbindung des weiteren Metalls wurde jedoch anstelle von Praseodymtrinitrat-Pentahydrat 0,71 g (0,0017 mol) Samariumtrinitrat Pentahydrat verwendet.

Der fertige Katalysator enthielt 1,1 Gew.-% Pd, 1,1 Gew.-% Au, 2,5 Gew.-% K und 0,16 Gew.-% Sm.

### Beispiel 3

Es wurde analog der Arbeitsweise nach Beispiel 1 vorgegangen, als Verbindung des weiteren Metalls wurde jedoch 0,7 g (0,0016 mol) Europiumtrinitrat-Pentahydrat verwendet.

Der fertige Katalysator enthielt 1,1 Gew.-% Pd, 1,1 Gew.-% Au, 2,5 Gew.-% K und 0,15 Gew.-% Eu.

### Beispiel 4

Es wurde analog der Arbeitsweise nach Beispiel 1 vorgegangen, als Verbindung des weiteren Metalls wurde jedoch 0,34 g (0,0008 mol) Neodymtrinitrat-Pentahydrat verwendet.

Der fertige Katalysator enthielt 1,1 Gew.-% Pd, 1,1 Gew.-% Au, 2,5 Gew.-% K und 0,07 Gew.-% Nd.

### Beispiel 5

Es wurde analog der Arbeitsweise nach Beispiel 1 vorgegangen, als Verbindung des weiteren Metalls wurde jedoch 0,3 g (0,0008 mol) Dysprosiumtrichlorid-Hexahydrat verwendet.

Der fertige Katalysator enthielt 1,1 Gew.-% Pd, 1,1 Gew.-% Au, 2,5 Gew. -% K und 0,08 Gew. -% Dy.

### Beispiel 6

Es wurde analog der Arbeitsweise nach Beispiel 5 vorgegangen, es wurden jedoch 0,6 g (0,0016 mol) Dysprosiumtrichlorid-Hexahydrat verwendet.

Der fertige Katalysator enthielt 1,1 Gew.-% Pd, 1,1 Gew.-% Au, 2,5 Gew.-% K und 0,16 Gew. -% Dy.

### Vergleichsbeispiel 1a

Es wurde wie in Beispiel 1 vorgegangen allerdings wurde auf den Zusatz des Salzes des weiteren Metalls zu der Kaliumtetrachloropalladat und Kaliumtetrachloroaurat enthaltenden Imprägnierlösung verzichtet.

Der fertige Katalysator enthielt 1,1 Gew.-% Pd, 1,1 Gew.-% Au und 2,5 Gew.-% K.

Die Austestung der nach den Beispielen 1 - 6 hergestellten erfindungsgemäßen Katalysatoren sowie des nach dem Vergleichsbeispiel 1a hergestellten bekannten Katalysators erfolgte in einem Berty-Reaktor. Die mittlere Manteltemperatur des Berty-Reaktors wurde so gewählt, daß ein konstanter Sauerstoffumsatz von 45 % beobachtet wurde.

Die Ergebnisse sind aus der Tabelle ersichtlich.

| Beispiel | Raum-Zeit-Ausbeute | CO₂-Selektivität |
|---|---|---|
| 1 | 793 | 8,97 |
| 2 | 780 | 9,23 |
| 3 | 802 | 8,79 |
| 4 | 726 | 8,50 |
| 5 | 733 | 9,0 |
| 6 | 722 | 9,3 |
| Vergleichsbeispiel 1a | 683 | 10,9 |

Raum-Zeit-Ausbeute in Gramm Vinylacetat pro Liter Katalysator und Stunde.

CO₂-Selektivität in %, bezogen auf die Menge umgesetztes Ethylen.

Überraschenderweise wurde gefunden, daß schon geringe Zusätze an den genannten weiteren Metallen zu den bekannten Palladium, Gold und Kalium enthaltenden Katalysatoren die CO₂-Selektivität und die Leistungsfähigkeit (Raum-Zeit-Ausbeute) dieser Katalysatoren in der Vinylacetatherstellung deutlich verbessern.

## Patentansprüche

1. Verfahren zur Herstellung von Vinylacetat in der Gasphase aus Ethylen, Essigsäure und Sauerstoff oder Sauerstoff enthaltenden Gasen an einem Katalysator, der 0,5-2,0 Gew.-% Palladium und/oder dessen Verbindungen, 0,2-1,3 Gew.-% Gold und/oder dessen Verbindungen sowie 0,3-10 Gew.-% Alkalimetallverbindungen auf einem Träger enthält, **dadurch gekennzeichnet, daß** der Katalysator zusätzlich 0,01-1 Gew.-% mindestens ein weiteres Metall, das aus der Gruppe ausgewählt wird, die aus Praseodym, Neodym, Promethium, Samarium, Europium, Gadolinium, Terbium, Dysprosium, Holmium, Erbium, Thulium, Ytterbium und Lutetium, Scandium, Yttrium und Lanthan besteht, und/oder dessen Verbindungen enthält, wobei sich die Prozentangaben auf die Metallgehalte, bezogen auf die Gesamtmasse des Katalysators, beziehen.

2. Verfahren nach Anspruch 1, **dadurch gekennzeichnet, daß** der Katalysator mindestens eine Kaliumverbindung enthält.

3. Verfahren nach Anspruch 1 oder 2, **dadurch gekennzeichnet, daß** der Katalysator 0,05 Gew.-% bis 0,5 Gew.-% des genannten weiteren Metalls, bezogen auf die Gesamtmasse des Katalysators, enthält.

4. Verfahren nach einem der Ansprüche 1 bis 3, **dadurch gekennzeichnet, daß** das genannte weitere Metall Praseodym, Samarium, Europium, Neodym oder Dysprosium ist.

5. Katalysator, der 0,5-2,0 Gew.-% Palladium und/oder dessen Verbindungen, 0,2-1,3 Gew.-% Gold und/oder dessen Verbindungen sowie 0,3-10 Gew.-% Alkalimetallverbindungen auf einem Träger enthält, **dadurch gekennzeichnet, daß** der Katalysator zusätzlich 0,01-1 Gew.-% mindestens ein weiteres Metall, das aus der Gruppe ausgewählt wird, die aus Praseodym, Neodym, Promethium, Samarium, Europium, Gadolinium, Terbium, Dysprosium, Holmium, Erbium, Thulium, Ytterbium und Lutetium, Scandium, Yttrium und Lanthan besteht, und/oder dessen Verbindungen enthält, wobei sich die Prozentangaben auf die Metallgehalte, bezogen auf die Gesamtmasse des Katalysators, beziehen.

6. Katalysator nach Anspruch 5, **dadurch gekennzeichnet, daß** der Katalysator mindestens eine Kaliumverbindung enthält.

7. Katalysator nach Anspruch 5 oder 6, **dadurch gekennzeichnet, daß** der Katalysator 0,05 Gew.-% bis 0,5 Gew.-% des genannten weiteren Metalls, bezogen auf die Gesamtmasse des Katalysators, enthält.

8. Katalysator nach einem der Ansprüche 5 bis 7, **dadurch gekennzeichnet, daß** das genannte weitere Metall Praseodym, Samarium, Europium, Neodym oder Dysprosium ist.

## Claims

1. Process for producing vinyl acetate in the gas phase from ethylene, acetic acid and oxygen or gases containing oxygen on a catalyst which contains 0.5-2.0 wt.% of palladium and/or its compounds, 0.2-1.3 wt.% of gold and/or its compounds and 0.3-10 wt.% of alkali metal compounds on a support, **characterised in that** the catalyst additionally contains 0.01-1 wt.% of at least one further metal which is selected from the group which consists of praseodymium, neodymium, promethium, samarium, europium, gadolinium, terbium, dysprosium, holmium, erbium, thulium, ytterbium and lutetium, scandium, yttrium and lanthanum, and/or its compounds, wherein the percentage details relate to the metal contents, based on the total mass of the catalyst.

2. Process according to claim 1, **characterised in that** the catalyst contains at least one potassium compound.

3. Process according to claim 1 or 2, **characterised in that** the catalyst contains 0.05 wt.% to 0.5 wt.% of the said further metal, based on the total mass of the catalyst.

4. Process according to one of claims 1 to 3, **characterised in that** the said further metal is praseodymium, samarium, europium, neodymium or dysprosium.

5. Catalyst which contains 0.5-2.0 wt.% of palladium and/or its compounds, 0.2-1.3 wt.% of gold and/or its compounds and 0.3-10 wt.% of alkali metal compounds on a support, **characterised in that** the catalyst additionally contains 0.01-1 wt.% of at least one further metal which is selected from the group which consists of praseodymium, neodymium, promethium, samarium, europium, gadolinium, terbium, dysprosium, holmium, erbium, thulium, ytterbium and lutetium, scandium, yttrium and lanthanum, and/or its compounds, wherein the percentage details relate to the metal contents, based on the total mass of the catalyst.

6. Catalyst according to claim 5, **characterised in that** the catalyst contains at least one potassium compound.

7. Catalyst according to claim 5 or 6, **characterised in that** the catalyst contains 0.05 wt.% to 0.5 wt.% of the said further metal, based on the total mass of the catalyst.

8. Catalyst according to one of claims 5 to 7, **characterised in that** the said further metal is praseodymium, samarium, europium, neodymium or dysprosium.

## Revendications

1. Procédé pour la fabrication d'acétate vinylique en phase gazeuse à partir d'éthylène, d'acide acétique et d'oxygène ou de gaz contenant de l'oxygène au niveau d'un catalyseur qui contient 0,5 à 2,0 % en poids de palladium et/ou de ses composés, 0,2 à 1,3 % en poids d'or et/ou de ses composés, ainsi que 0,3 à 10 % en poids de composés de métaux alcalins sur un support, **caractérisé en ce que** le catalyseur se compose par ailleurs de 0,01 à 1 % en poids d'un autre métal au moins, lequel est choisi dans le groupe se composant de praséodyme, de néodyme, de prométhium, de samarium, d'europium, de gadolinium, de terbium, de dysprosium, d'holmium, d'erbium, de thulium, d'ytterbium et de lutétium, de scandium, d'yttrium et de lanthane et/ou de leurs composés, les données en pourcentage se rapportant à la teneur en métal par rapport à la masse totale du catalyseur.

2. Procédé selon la revendication 1, **caractérisé en ce que** le catalyseur contient au moins un composé potassium.

3. Procédé selon la revendication 1 ou 2, **caractérisé en ce que** le catalyseur comprend de 0,05 % à 0,5 % en poids d'autres métaux cités, rapportés à la masse totale du catalyseur.

4. Procédé selon l'une quelconque des revendications 1 à 3, **caractérisé en ce que** l'autre métal cité est le praséodyme, le samarium, l'europium, le néodyme ou le dysprosium.

5. Catalyseur qui contient 0,5 à 2,0 % en poids de palladium et/ou de ses composés, 0,2 à 1,3 % en poids d'or et/ou de ses composés, ainsi que 0,3 à 10 % en poids de composés de métaux alcalins sur un support, **caractérisé en ce que** le catalyseur comprend par ailleurs 0,01 à 1 % en poids d'un autre métal au moins, lequel est choisi dans le groupe se composant de praséodyme, de néodyme, de prométhium, de samarium, d'europium, de gadolinium, de terbium, de dysprosium, d'holmium, d'erbium, de thulium, d'ytterbium et de lutétium, de scandium, d'yttrium et de lanthane, et/ou de leurs composés, les données en pourcentage se rapportant à la teneur en métal par rapport à la masse totale du catalyseur.

6. Catalyseur selon la revendication 5, **caractérisé en ce que** le catalyseur contient au moins un composé potassium.

7. Catalyseur selon la revendication 5 ou 6, **caractérisé en ce que** le catalyseur comprend de 0,05 % à 0,5 % en poids d'autres métaux cités, rapportés à la masse totale du catalyseur.

8. Catalyseur selon l'une quelconque des revendications 5 à 7, **caractérisé en ce que** l'autre métal cité est le praséodyme, le samarium, l'europium, le néodyme ou le dysprosium.
